# EUROPÄISCHE PATENTSCHRIFT

(11) **EP 3 280 699 B1**
(45) Veröffentlichungstag und Bekanntmachung des Hinweises auf die Patenterteilung: **10.07.2019**
(21) Anmeldenummer: 16717855.7
(22) Anmeldetag: 04.04.2016
(51) Int. Cl.: C07C 303/40, C07C 311/51

(54) **VERFAHREN ZUR HERSTELLUNG VON ACYLSULFAMOYLBENZAMIDEN**
METHOD FOR PREPARING ACYLSULFAMOYLBENZAMIDES
PROCÉDÉ DESTINÉ À LA PRODUCTION D'ALLIAGES D'ACYLSULFAMOYLBENZAMIDES

(30) Priorität: 08.04.2015 EP 15162810
(43) Veröffentlichungstag der Anmeldung: 14.02.2018
(73) Patentinhaber: Bayer CropScience Aktiengesellschaft, 40789 Monheim am Rhein (DE)
(72) Erfinder: FARIDA, Taraneh, 50259 Pulheim-Geyen (DE); ESSER, Michael, 51379 Leverkusen (DE); STAKEMEIER, Hubertus, 51467 Bergisch-Gladbach (DE)
(74) Vertreter: BIP Patents
(86) Internationale Anmeldenummer: PCT/EP2016/057322
(87) Internationale Veröffentlichungsnummer: WO 2016/162299

(56) Entgegenhaltungen:
- WO-A1-2005/000797
- WO-A1-2015/052156
- CN-A- 101 838 227

## Beschreibung

Die Erfindung betrifft ein Verfahren zur Herstellung von Acylsulfamoylbenzamiden, inbesondere betrifft die Erfindung ein verbessertes Verfahren zur Herstellung von *N*-[4-(cyclopropylcarbamoyl)phenyl-sulfonyl]-2-methoxybenzamid.

*N*-[4-(cyclopropylcarbamoyl)phenylsulfonyl]-2-methoxybenzamid (oder alternativ: *N*-[4-(cyclopropylcarbamoyl)phenylsulfonyl]-o-anisamide) wird auch mit dem *common name* Cyprosulfamide bezeichnet. Cyprosulfamide wird in der Landwirtschaft in Kombination mit einem Herbizid, bzw. mit mehreren unterschiedlichen Herbiziden, als Safener eingesetzt. Ein Safener dient zur Verbesserung der Selektivität der eingesetzten Herbizide gegenüber den Nutzpflanzen der jeweils behandelten Kultur. Der Begriff der Selektivität bezeichnet die Nutzpflanzenverträglichkeit eines Herbizids.

Dokument WO 99/16744 offenbart Acylsulfamoylbenzamid-Derivate sowie deren Herstellung und Verwendung als Safener. Jedoch betreffen die in WO 99/16744 offenbarten Herstellungsverfahren den Labormaßstab und erwiesen sich für die industrielle Herstellung der Verbindungen als nicht besonders geeignet.

Dokument CN 101 838 227 A offenbart Konstitutionsisomere von Cyprosulfamide sowie ein alternatives Verfahren zu deren Herstellung.

Ein zweistufiges Verfahren, das zur Herstellung von Acylsulfamoylbenzamiden im industriellen Maßstab entwickelt worden ist, ist aus Dokument WO 2005/000797 A1 bekannt.

Ein weiteres zweistufiges Verfahren, das auch die Herstellung einer für die Cyprosulfamide-Synthese erforderlichen Amidchlorid-Vorstufe umfasst, und das alternativ auch als einstufiges Verfahren (d.h. ohne vorherige Isolierung der Vorstufe (Intermediat) im Rahmen einer sog. Eintopfsynthese angewendet werden kann, wird in der internationalen Patentanmeldung PCT/EP2014/071388 (WO 2015/052156 A1) beschrieben.

Das in der vorgenannten Patentanmeldung WO 2015/052156 A1 beschriebene Verfahren eignet sich ebenfalls zur Herstellung von Acylsulfamoylbenzamiden (la) im industriellen Maßstab, wobei kein organisches Lösungsmittel eingesetzt wird. D.h. die Umsetzung (Reaktion) eines Amidchlorids mit einem sekundären Amin als Edukte erfolgt in einer wässrigen Lösung ohne den Einsatz organischer Lösungsmittel.

Der Anmeldetag der WO 2015/052156 A1 liegt vor dem Anmeldetag der vorliegenden Anmeldung, wobei die Patentanmeldung WO 2015/052156 A1 am Anmeldetag der vorliegenden Anmeldung noch nicht offengelegt war.

### Aminierung - Option I

Bei einer nach Schotten-Baumann bekannten Verfahrensoption (Option I) enthält die Vorlage die beiden Edukte, d.h. das Amidchlorid der Formel (II) und das sekundäre Amin der Formel RRNH während im Reaktionsverlauf wässrige Alkalilauge (Natronlauge oder Kalilauge) zugetropft wird.

Ohne ein organisches Lösungsmittel ist die gemäß Option I bekannte Reaktionsführung jedoch nicht möglich. Ohne organische Lösungsmittel bildet das Reaktionsgemisch einen festen kaum rührbaren Block, da massemäßig wenig flüssiges Amin auf viel festes Amidchlorid trifft. Aus ökologischen und aus ökonomischen Gründen wird jedoch angestrebt den Einsatz von organischen Lösungsmitteln bei der großtechnischen Produktion zu vermeiden.

### Aminierung - Option II

Der Einsatz von Wasser als Verdünnungsmittel wäre bei der Verfahrensführung gemäß Option I prinzipiell möglich, jedoch würde das Amidchlorid der Formel (II) in einer unerwünschte Nebenreaktion hydrolisieren und dadurch zu schlechteren Ausbeuten führen.

Bei einer zweiten nach Schotten-Baumann bekannten alternativen Reaktionsführung (Option II) wird das sekundäre Amin der Formel RRNH in einer Hilfsbase (z.B. NaOH oder KOH) vorgelegt, während das Amidchlorid der Formel (II) als Feststoff zudosiert wird.

Die gleichzeitige Vorlage der beiden Basen ist für den Ablauf der Reaktion nicht störend, weil Amine stärkere Nucleophile sind als die Hydroxidionen der Alkalilauge, d.h. die Hydroxidionen der Alkalilauge beeinflussen die Reaktion nicht, sondern dienen allein der Neutralisation der freigesetzten Säure.

Die Anwendung der vorgenannten Option II zur Herstellung von Verbindungen der nachstehend genannten Formel (la) im großtechnischen Maßstab erwies sich dennoch als noch nicht ausreichend effizient. Der pH-Wert des Reaktionsgemischs bei der Reaktionsführung gemäß Option II liegt im Bereich von pH 13 bis 14.

### Aminierung - Option III

Eine weitere alternative Reaktionsführung (Option III) beschreibt die oben genannte internationale Anmeldung PCT/EP2014/071388 (WO 2015/052156 A1) von Farida et al.. Bei dieser Option III erfolgt die Vorlage eines sekundären Amins zusammen mit dem Amidchlorid der nachstehend genannten Formel (II) in einer wässrigen NaOH-Lösung.

Überraschender Weise werden bei der Verfahrensführung nach Option III zur Herstellung von Acylsulfamoylbenzamiden, auch ohne den Einsatz eines organischen Lösungsmittels, hohe Ausbeuten erzielt.

Gegenüber den vorbekannten Reaktionen (Optionen I und II) zeichnet sich das Verfahren gemäß der Patentanmeldung PCT/EP2014/071388 (WO 2015/052156 A1) (Option III) somit dadurch aus, dass zur Herstellung der Zielverbindung der Formel (la) kein organisches Lösungsmittel eingesetzt wird, d.h. die Reaktion erfolgt allein in einer wässrigen Lösung.

Im Rahmen der vorliegenden Erfindung, welche bei der Implementierung des in der Patentanmeldung PCT/EP2014/071388 (WO 2015/052156 A1) offenbarten Verfahrens im chemischen Betrieb (d.h. der großtechnischen Herstellung) gemacht wurde, konnte jedoch erkannt werden, dass sich bei vollständiger Vorlage der stöchiometrischen Menge eines sekundären Amins der Formel RRNH, bereits zum Beginn der Reaktion, eine unerwünschte Erhöhung des pH-Werts ergeben kann.

Weiterhin wurde erkannt, dass die unerwünschte Erhöhung des pH-Werts, nach Zugabe des Amidchlorids (II) im weiteren Reaktionsverlauf, die Entstehung der entsprechenden Säure des Amidchlorids (II) bewirkt. Die unerwünschte Säure des, Amidchlorids (II) ist die 4-[[(2-Methoxybenzoyl)amino]sulfonyl]benzoesäure, die in Gegenwart der Alkliionen (Na-Ionen oder K-Ionen) das entsprechende Salz bildet. Dieses Salz reagiert mit dem sekundären Amin der Formel RRNH nicht mehr in ausreichendem Maß zum Endprodukt. Dies führt zu hohen Ausbeuteverlusten.

Aufgrund der großen Herstellungsmengen, die üblicherweise bei der großtechnischen Anwendung eines Verfahrens angestrebt werden, können aus wirtschaftlichen und auch aus ökologischen Gründen bereits vermeintlich geringfügige Verbesserungen eines Verfahrens sehr relevant sein.

Vor diesem Hintergrund besteht die Aufgabe der Erfindung in der Bereitstellung eines verbesserten alternativen Verfahrens zur Herstellung von Acylsulfamoylbenzamiden, wobei sich das alternative Verfahren durch hohe Robustheit bei zugleich hohen Ausbeuten auszeichnen soll.

Gelöst wird die Aufgabe durch ein Verfahren zur Herstellung von Verbindungen der Formel (Ia) 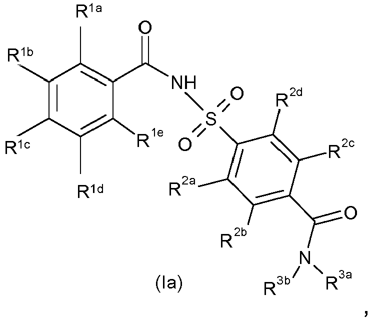 worin
R^{1a} bis R^{1e} und R^{2a} bis R^{2d} jeweils unabhängig voneinander ausgewählt sind aus der Gruppe bestehend aus Wasserstoff, Halogen, (C₁-C₆)-Alkyl, (C₁-C₆)-Halogenalkyl, (C₁-C₆)-Alkoxy, (C₃-C₇)-Cycloalkyl, (C₁-C₆)-Alkylthio, (C₃-C₇)-Cycloalkylthio, S(O)_{q}-(C₁-C₆)-Alkyl mit q = 0, 1 oder 2, (C₁-C₆)-Alkylcarbonyl, -CO-aryl, Cyano und Nitro oder in welcher jeweils zwei benachbarte Reste R^{1a} bis R^{1e} einen -O-CH₂CH₂- Rest bilden, und
R^{3a} ausgewählt ist aus der Gruppe bestehend aus Wasserstoff und den nachgenannten Resten (C₁-C₆)-Alkyl, (C₁-C₆)-Halogenalkyl, (C₃-C₇)-Cycloalkyl, (C₁-C₆)-Alkoxy, (C₁-C₆)-Alkylthio, (C₃-C₇)-Cycloalkylthio, -(CH2)p-Heterocyclyl, wobei diese jeweils unsubstituiert sind oder substituiert sind durch einen oder mehrere Substituenten ausgewählt aus der Gruppe bestehend aus Halogen, (C₁-C₆)-Alkyl, (C₁-C₆)-Halogenalkyl, (C₁-C₆)-Alkoxy, Cyano und Nitro, und
R^{3b} ausgewählt ist aus der Gruppe bestehend aus Wasserstoff und den nachgenannten Resten (C₁-C₆)-Alkyl, (C₂-C₆)-Alkenyl, (C₂-C₆)-Alkinyl, (C₁-C₆)-Alkoxy, (C₂-C₆)-Alkenyloxy, -(CH2)p-Heterocyclyl, wobei diese jeweils unsubstituiert sind oder substituiert sind durch einen oder mehrere Substituenten ausgewählt aus der Gruppe bestehend aus Halogen, (C₁-C₄)-Alkoxy und (C₁-C₄)-Alkylthio, oder R^{3a} und R^{3b} gemeinsam mit dem verbindenden Stickstoffatom einen 3- bis 8-gliedrigen, gesättigten oder ungesättigten Ring ausbilden
durch Umsetzung einer Verbindung der Formel (II) 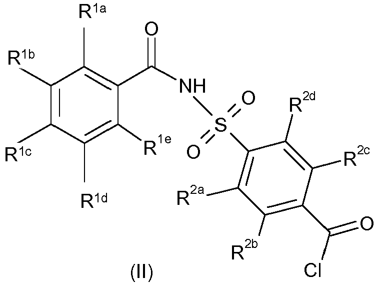 mit einem Amin der Formel R^{3a}R^{3b}NH, worin die Reste R^{3a} und R^{3b} wie oben definiert sind, in wässriger Lösung, wobei zur Regulation des pH-Werts
- bei Reaktionsbeginn nur ein Teil des Amins der Formel R^{3a}R^{3b}NH in Wasser vorgelegt wird, und
- während des Reaktionsverlaufs die verbleibende Menge des Amins der Formel R^{3a}R^{3b}NH dem Reaktionsgemisch in einem oder in mehreren Schritten zugegeben wird.

Das Verfahren gemäß der vorliegenden Erfindung baut auf der Verwendung von Wasser als Lösungsmittel gemäß der oben genannten Option III auf, wobei die Reaktionsvorlage beim erfindungsgemäßen Verfahren keine Alkalilauge enthält.

Gegenüber dem Stand der Technik gemäß Option I und II zeichnet sich das erfindungsgemäße Verfahren somit dadurch aus, dass als Lösungsmittel Wasser und keine organischen Lösungsmittel eingesetzt werden.

Gegenüber dem in der internationalen Patentanmeldung PCT/EP2014/071388 (WO 2015/052156 A1) beschriebenen Verfahren ist das erfindungsgemäße Verfahren deshalb neu, weil die Vorlage der Reaktion beim erfindungsgemäßen Verfahren keine Alkalilauge, z.B. Natronlauge, enthält. Statt dessen wird beim erfindungsgemäßen Verfahren eine geringe Menge des Amins der Formel R^{3a}R^{3b}NH in Wasser vorgelegt, wobei die Reste R^{3a}R^{3b} wie in der vorgenannten Formel (Ia) definiert sind.

Die verbleibende Aminmenge wird dem Reaktionsgemisch bevorzugt während (gleichzeitig mit) der Zugabe des Amidchlorids der Formel (II) zugegeben. D.h. die Zugabe der verbleibenden Aminmenge erfolgt bevorzugt parallel zur Zugabe des Amidchlorids.

Die frühe Regulation des pH-Wertes im vorgelegten Reaktionsgemisch durch Dosierung der Aminzugabe gemäß der Lehre des erfindungsgemäßen Verfahrens bewirkt, im Vergleich zu den aus WO 99/16744 und aus WO 2005/000797 A1 vorbekannten Verfahren, eine erheblich Verbesserung der Acylsulfamoylbenzamid-Ausbeute.

Die Besonderheit dieses einleitenden Verfahrensschritts, d.h. die anfängliche Regulation des pH-Wertes bei der Herstellung von Cyprosulfamide, basiert auf der Beachtung des Umstandes, dass die chemische Struktur des Eduktes der Formel (II) 4-[[(2-Methoxybenzoyl)amino]sulfonyl]-benzoylchlorid ein acides Proton und die chemische Struktur des Endprodukts Cyprosulfamide sogar zwei acide Protonen aufweist. Aus diesem Grund ist die Hydrolyseempfindlichkeit von beiden Verbindungen als hoch einzustufen. Allerdings ist diese Hydrolyseempfindlichkeit stark vom pH-Wert der Lösung, in welcher die genannten Verbindungen vorliegen, abhängig.

Erfindungsgemäß wurde erkannt, dass bereits durch die Vorlage von basischem Amin der Formel R^{3a}R^{3b}NH die Hydrolyseempfindlichkeit des Eduktes der Formel (II) 4-[[(2-Methoxybenzoyl)amino]sulfonyl]-benzoylchlorid (und die des entstehenden Cyprosulfamides) in einem unerwarteten Ausmaß abgeschwächt werden kann.

Die Vermeidung, bzw. Abschwächung der Hydrolyse-Reaktion ist von Vorteil, weil dadurch die Entstehung der 4-[[(2-Methoxybenzoyl)amino]sulfonyl]benzoesäure (aufgrund der Gegenwart von Alkaliionen in der Reaktionslösuing als reaktionsträges Salz vorliegend), unterbunden wird und so in der Folge die Erzielung höherer Cyprosulfamide-Ausbeuten möglich ist.

Die Erfindung betrifft somit die Optimierung der Ausbeute sowie die Verbesserung der Robustheit des Verfahrens. Aufgrund der in der industriellen Fertigung vergleichsweise großen Produktmengen ist in wirtschaftlicher Hinsicht bereits auch eine nur geringfügige Verbesserung der Ausbeute von großer Bedeutung. Auch eine Verbesserung der Reinheit des Produktes kann wirtschaftlich von großer Bedeutung sein.

Daher ist die Verbesserung eines großtechnisch durchführbaren Verfahrens durch Variation aller Reaktionsparameter in der industriellen Wirkstoffherstellung grundsätzlich ein beständiges Bemühen. Jedoch handelt sich, bei der Variation der Parameter oft, trotz des systematischen Vorgehens, nicht um ein rein routinemäßiges Erproben.

Gerade der unerwartet starke Einfluss der anfänglichen (sowie der späteren) pH-Regulation durch Dosierung der Aminzugabe auf eine chemische Reaktion kann oft nur rückblickend erkannt und begründet werden.

Bevorzugt ist das erfindungsgemäße Verfahren zur Herstellung von Verbindungen der Formel (Ia) worin die Reste R^{1a} bis R^{1e} und R^{2a} bis R^{2d} unabhängig voneinander ausgewählt sind aus der Gruppe bestehend aus
Wasserstoff, Fluor, Chlor, Brom und
(C₁-C₆)-Alkoxy.

Besonders bevorzugt ist ein Verfahren zur Herstellung von Verbindungen der Formel (Ia) worin R^{1a} ausgewählt ist aus der Gruppe bestehend aus unsubstituierten (C₁-C₄)-Alkoxyresten und R^{1b} bis R^{1e} sowie R^{2a} bis R^{2d} jeweils Wasserstoff sind.

Ganz besonders bevorzugt ist ein Verfahren zur Herstellung von Verbindungen der Formel (Ia) worin R^{1a} für Methoxy (-O-CH₃) steht, und R^{1b} bis R^{1e} sowie R^{2a} bis R^{2d} jeweils für Wasserstoff stehen.

Am meisten bevorzugt ist ein Verfahren zur Herstellung von Verbindungen der Formel (la) worin
- R^{1a} für Methoxy (-O-CH₃) sowie R^{1b} bis R^{1e} sowie R^{2a} bis R^{2d} jeweils für Wasserstoff stehen, und in dem
- Amin der Formel R^{3a}R^{3b}NH der Rest R^{3a} für Cycloproypl und R^{3b} für Wasserstoff steht.

Das bei dem vorgenannten am meisten bevorzugten Verfahren entstehende Produkt ist Cyprosulfamide und hat die Formel (Ib) 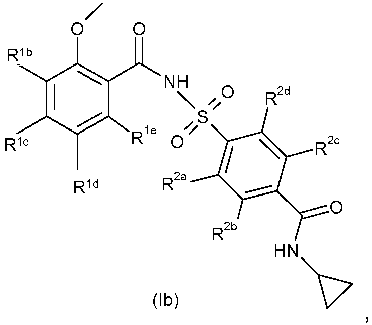 worin
die Reste R^{1b} bis R^{1e} sowie R^{2a} bis R^{2d} jeweils Wasserstoff sind.

In einer bevorzugten Ausführungsform der Erfindung ist vorgesehen, dass der pH-Wert der Vorlage bei Reaktionsbeginn maximal 11,5 (pH ≤ 11,5) beträgt. Dies wird durch die Vorlage einer entsprechenden Menge des Amins der Formel R^{3a}R^{3b}NH in Wasser erreicht, Erst nach dieser anfänglichen Einstellung des pH-Werts in der Reaktionsvorlage auf pH ≤ 11,5 erfolgt die Zugabe des Eduktes der Formel (II).

Die Zugabe des als Feststoff vorliegenden Amidchorids, d.h. des Eduktes der Formel (II), zur wässrigen Aminlösung, welche die Reaktionsvorlage bildet, kann quantitativ erfolgen. Bevorzugt erfolgt die Zugabe des Amidchlorids (Säurechlorids), jedoch dosiert, d.h. in mehreren Schritten. Besonders bevorzugt ist die sich über einen Zeitraum von ca. 4 Stunden erstreckende Zugabe in 20 bis 30 Schritten (Portionen). Im Rahmen der technischen Herstellung ist die kontinuierliche Zugabe des Amidchlorids mittels einer Dosierschnecke ganz besonders bevorzugt.

Die Zugabe des Amins der Formel R^{3a}R^{3b}NH erfolgt gemäß der Lehre der vorliegenden Erfindung insgesamt in mindestens zwei Schritten.

Der erste Zugabeschritt betrifft die Aminzugabe bei Reaktionsbeginn, mit dem Ziel der Einstellung pH-Wertes in der Reaktionsvorlage auf pH ≤ 11,5. Die Zugabe der verbleibenden Menge des Amins der Formel R^{3a}R^{3b}NH erfolgt entweder in einem Schritt (2-stufige Aminzugabe) oder in mehreren Schritten (mindestens 3-stufige Aminzugabe).

Die Zugabe der verbleibenden Menge des Amins der Formel R^{3a}R^{3b}NH in mehreren Schritten (d.h. mindestens 3-stufige Aminzugabe) umfasst natürlich auch die dosierte Zugabe des Amins in sehr kleinen Mengen, z.B. durch Zugabe des Amins zum Reaktionsgemisch unter Einsatz einer oder mehrerer Zuleitungen.

Ganz besonders bevorzugt in der technischen Herstellung ist die kontinuierliche Zugabe des Amins in sehr vielen Schritten, z.B. durch das pH-geregelte Zutropfen oder das Zupumpen des Amins zu Reaktionsgemisch.

In einer besonders bevorzugten Ausführungsform der Erfindung ist vorgesehen, dass die Zugabe der bei Reaktionsbeginn nicht vorgelegten Aminmenge des Amins der Formel R^{3a}R^{3b}NH in mehreren Schritten erfolgt, so dass der pH-Wert während der Zugabe im weiteren Reaktionsverlauf im Bereich von 7 bis 10,5 liegt (pH = 7 - 10,5).

Die pH-geregelte Zugabe des Amins der Formel R^{3a}R^{3b}NH zum Reaktionsgemisch im Rahmen der mindestens 3-stufigen Aminzugabe erfolgt bevorzugt bis 1,1 Äquivalente, bezogen auf die vorgelegten Menge an Amidchlorid (Säurechlorid), erreicht sind.

Es liegt im Rahmen der Erfindung, dass die nachfolgende pH-geregelte Aminzugabe durch Zutropfen oder durch Zupumpen erfolgt.

Die pH-geregelte Zugabe des Amins der Formel R^{3a}R^{3b}NH zum Reaktionsgemisch erfolgt bevorzugt so, dass der pH-Wert während der Zugabe im weiteren Reaktionsverlauf im Bereich von 9 bis 10,5 (pH = 9 bis 10,5), bzw. ganz besonders bevorzugt im Bereich von 9,0 bis 9,6 (pH = 9,0 bis 9,6), liegt.

Ein pH der über dem Wert von 10,5 liegt, bewirkt bereits eine unerwünschte Carbonsäurebildung und bei pH 13 liegen bereits ca. 5% des Amidchlorids der Formel (II) als Carbonsäure vor. Dies bewirkt eine unerwünschte Verminderung des Ausbeute des Produktes der Formel (Ia).

In einer ganz besonders bevorzugten Ausführungsform der Erfindung ist der zusätzliche Einsatz einer Base, ausgewählt aus der Gruppe bestehend aus NaOH, KOH, Ca(OH)₂ und der Gruppe der tertiären Amine, insbesondere Triethylamin, zur Regulation des pH-Werts im Reaktionsgemisch (pH-Regulation) vorgesehen, wobei der pH-Wert des Reaktionsgemischs durch die Zugabe der Base im Bereich von 8 bis 10 stabilisiert werden soll. Insbesondere soll durch Zugabe von NaOH ein Abfallen des pH-Wertes unter den pH-Wert 8 vermieden werden, d.h. der durch NaOH-Zugabe zum Abschluss der Reaktion angestrebte pH-Wert ist ≥ 8.

Die zusätzliche Base, ausgewählt aus der Gruppe bestehend aus NaOH, KOH, Ca(OH)₂ und der Gruppe der tertiären Amine, wird also nicht der Reaktionsvorlage zugegeben, sondern die zusätzliche Base wird dem Reaktionsgemisch, das bereits auch das gewünschte Edukt enthält, zugegeben.

Der zusätzliche Einsatz einer Base, ausgewählt aus der Gruppe bestehend aus NaOH, KOH, Ca(OH)₂ und der Gruppe der tertiären Amine, erfolgtwährend der Reaktion, bevorzugt nach vollständiger Zugabe der Amins und nach Zugabe von 20 % des Amidchlorids.. Alternativ kann die Zugabe der Base auch als abschließende Zugabe, d.h. nach vollständiger Zugabe des Amidchlorids, erfolgen.

Am meisten bevorzugt ist der Einsatz von NaOH zur Einstellung eines pH-Werts, der im Bereich von 9 bis 10 (pH = 9 bis 10) liegt.

Es hat sich gezeigt, dass die abschließende Zugabe einer geringen Menge eines organischen Lösungsmittels, z.B. von Toluol, die Rührbarkeit (und Pumpbarkeit) des als schwer pumpbare Suspension vorliegenden Produktes erleichtert.

Somit umfasst eine bevorzugte Form der Durchführung des erfindungsgemäßen Verfahrens zusammen die nachfolgend genannten allgemeinen Verfahrensschritte:
- Vorlage von Wasser und einer geringen Menge Cyclopropylamin (=CPA), so dass pH bei max. 11,5 liegt, und die bevorzugt parallele
- Zugabe des Amidchlorids (feste Menge/h) und des Amins bei pH 7-10,5) bis 1,1 Äquivalente bezogen auf Amidchlorid erreicht sind, sowie die
- abschließende oder parallele Zugabe von Natronlauge zur weiterlaufenden Säurechlorid-Dosierung.

Allein zur Stützung der Ausführbarkeit des erfindungsgemäßen Verfahrens, das auf die verbesserte Herstellung von Acylsulfamoylbenzamiden, inbesondere N-[4-(cyclopropylcarbamoyl)phenyl-sulfonyl]-2-methoxybenzamid, gerichtet ist, wird nachfolgend auch auf eine spezifische Möglichkeit der Herstellung des Eduktes der Formel (II), d.h. 4-[[(2-Methoxybenzoyl)amino]sulfonyl]benzoylchlorid Bezug genommen.

Ein verbessertes Verfahren zur Herstellung des Eduktes der Formel (II) ist in der internationalen Patentanmeldung PCT/EP2014/071388 (WO 2015/052156 A1) beschrieben. Das vorgenannte Verfahren betrifft die Herstellung von4-[[(Benzoyl)-amino]sulfonyl]benzoylchloriden der Formel (II), 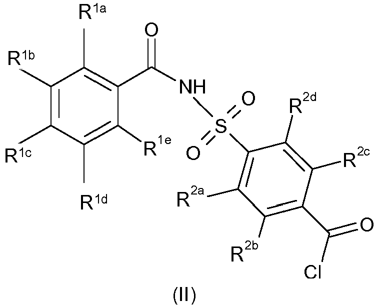 in welcher die Reste R^{1a} bis R^{1e} und die Reste R^{2a} bis R^{2d} jeweils unabhängig voneinander ausgewählt sind aus der Gruppe bestehend aus Wasserstoff, Halogen, (C₁-C₆)-Alkyl, (C₁-C₆)-Halogenalkyl, (C₁-C₆)-Alkoxy, (C₃-C₇)-Cycloalkyl, (C₁-C₆)-Alkylthio, (C₃-C₇)-Cycloalkylthio, S(O)_{q}-(C₁-C₆)-Alkyl mit q = 0, 1 oder 2, (C₁-C₆)-Alkylcarbonyl, -CO-aryl, Cyano und Nitro oder in welcher jeweils zwei benachbarte Reste R^{1a} bis R^{1e} einen -O-CH₂CH₂- Rest bilden, ausgehend von einer Verbindung der Formel (III) 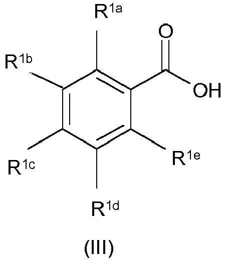 worin die Reste R^{1a} bis R^{1e} wie oben definiert sind, und einer Verbindung der Formel (IV) 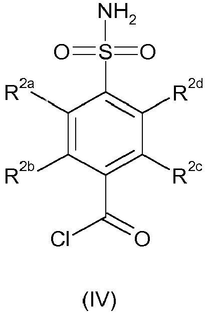 worin die Reste R^{2a} bis R^{2d} wie oben definiert sind, wobei die Verbindungen der Formel (III) und (IV) als Edukte
- in einem Lösungsmittel, welches ausgewählt ist aus der Gruppe der aprotischen polaren Lösungsmittel, oder
- in einer Lösungsmittelzusammensetzung, welche mindestens ein Lösungsmittel enthält, das ausgewählt ist aus der Gruppe der aprotischen polaren Lösungsmittel,
umgesetzt werden.

Bevorzugt anwendbar ist das vorgenannte Verfahren zur Herstellung von Verbindungen der Formel (II), in welchen die Reste R^{1a} bis R^{1e} und R^{2a} bis R^{2d} unabhängig voneinander ausgewählt sind aus der Gruppe bestehend aus Wasserstoff, Fluor, Chlor, Brom und
(C₁-C₆)-Alkoxy, wobei
das Verfahren besonders bevorzugt anwendbar ist zur Herstellung von Verbindungen der Formel (II) in welchen der Rest R^{1a} ein unsubstituierter (C₁-C₄)-Alkoxyrest ist.

Besonders bevorzugt ist die Anwendung des vorgenannten Verfahrens zur Herstellung von 4-[[(2-Methoxybenzoyl)amino]sulfonyl]benzoylchlorid, d.h. die erfindungsgemäße Umsetzung jener Verbindung der Formel (II), in welcher der Rest R^{1a} für Methoxy (-O-CH₃) steht und zugleich die Reste R^{1b} bis R^{1e} jeweils alle für Wasserstoff (H) stehen, ist besonders bevorzugt.

Nachfolgend wird auch erläutert, dass beim Einsatz der üblicherweise eingesetzten Lösungsmittel Toluol und Chlorbenzol zur Herstellung von *N*-[4-(cyclopropylcarbamoyl)phenyl-sulfonyl]-2-methoxybenzamiden eine höhere Dimerbildung festzustellen ist, als bei Einsatz eines aprotischen polaren Lösungsmittels. Die Vorteile des Einsatzes von aprotisch polaren Lösungsmitteln ergeben sich im Überblick aus der untenstehenden Tabelle 1 und deren nachfolgenden Analyse.

Es ist festzustellen, dass durch den Austausch der Lösungsmittel gegen ein aprotisch polares Lösungsmittel gleichzeitig auch verschiedene mit der Dimerbildung verbundene Probleme vermieden werden können.

Zu diesen Folgeproblemen gehören Filtrationsprobleme auf der Amidchlorid-Stufe, d.h. die Filtration der sich bei der Herstellung von 4-[[(2-Methoxybenzoyl)amino]sulfonyl]-benzoylchlorid ergebenden Reaktionslösung erweist sich in der Praxis als erschwert. Ein weiteres Problem ist die Verminderung der Ausbeute sowohl für die Stufe der Amidchloridherstellung als auch für die Stufe der Cyprosulfamideherstellung und die gleichzeitige Verschlechterung der Qualität, d.h. der Reinheit, beider Produkte.

Die genannten Probleme können durch den Einsatz von aprotisch polaren Lösungmitteln vermieden werden.

Aprotisch polare (dipolare) Lösungsmittel sind chemische Verbindungen, die sich dadurch auszeichnen, dass sie keine Protonen abspalten und zugleich polar sind.

Formal werden Carbonsäureester in der Literatur zum Teil, trotz ihrer Polarität, auch der Gruppe der aprotisch unpolaren Lösungsmittel zugeordnet. Im Zusammenhang mit der vorliegenden Erfindung wird daher aus Gründen der Klarheit festgestellt, dass im Fall der vorliegenden Erfindung Carbonsäureester, insbesondere die Ester der Propionsäure und der Essigsäure, wie z.B. Isopropylacetat, zur Gruppe der aprotischen polaren Lösungsmittel gezählt werden. Das Lösungsmittel Chlorbenzol dagegen ist, trotz seines hohen Dipolmoments, hydrophob in Wasser und somit kaum löslich, d.h. Chlorbenzol ist unpolar. Auch Toluol gehört zur Gruppe der unpolaren Lösungsmittel.

Festzustellen ist, dass gerade aprotische und zugleich polare Lösungsmittel bei der Amidchlorid-Herstellung die Bildung von Dimeren unterdrücken, so dass durch die verbesserte Reaktionsführung Verbindungen der Formel (II) mit höheren Ausbeuten gewonnen werden können.

Die aprotisch polaren Verbindungen, die erfindungsgemäß als Lösungsmittel geeignet sind, müssen chemisch stabil sowie destillierbar sein und sollten außerdem eine Molekülmasse (Molekulargewicht) von unter 200 aufweisen. Aufgrund der Obergrenze des Molekulargewichts sind diese Lösungsmittel durch einen vergleichsweise niedrigen Siedepunkt charakterisiert. So wird durch die Auswahl der in Frage kommenden Lösungsmittel zugleich eine Obergrenze für die Reaktionstemperatur festgelegt. Diese Temperaturobergrenze bewirkt zugleich eine Sicherungsfunktion und stellt bei der Umsetzung des Herstellungsverfahrens im technischen sowie im industriellen Maßstab im Hinblick auf die Reaktionsführung einen zusätzlichen Vorteil dar.

**TABELLE 1**

| Tabellarischer Vergleich der Ausbeuten aufgrund HPLC-Analytik bei Einsatz verschiedener Lösungsmittel zur Herstellung von Verbindungen der Formel (II), d.h. zur Herstellung von 4-[[(Benzoyl)amino]sulfonyl]benzoylchloriden. | | | | | | | | | | |
|---|---|---|---|---|---|---|---|---|---|---|
| Exp. Nr. | Lösungsmittel | Chlorierungsmittel | Reaktionszeit | | Auswaage | HPLC | Ausbeute | | Dimeres * | |
| | | Thionylchlorid | Reaktion | Nachrühren | | | Isoliert | Abwasser | Feststoff | |
| | | Eq mol | h | h | g | Gew.% | %d.Th. | %d.Th. | FI.% | %d.Th. |
| A1 | Isopropylacetat | 2,5 | 3,5 (80-90 °C) | 60 (90 °C) | 180,2 | 99,5 | 91,4 | 2,9 | 0,08 | 0,14 |
| A2 | Isopropylacetat | 2,5 | 7 (80-90 °C) | 0 | 190,4 | 98,4 | 95,0 | 1,0 | 0,10 | 0,18 |
| A3 | Isopropylacetat | 2,5 | 7 (80-90 °C) | 0 | 192,3 | 96,5 | 95,7 | 1,5 | 0,10 | 0,19 |
| A4 | Isopropylacetat | 2,5 | 7 (80-90 °C) | 0 | 192,7 | 99,1 | 98,4 | 1,3 | 0,35 | 0,67 |
| | | | | | | | | | | |
| B1 | Toluol | 2,5 | 5 (85 °C) | 1 (85 °C) | 162,5 | 96,4 | 88,6 | 3,1 | 1,05 | 1,85 |
| B2 | Toluol | 2,5 | 7 (85 °C) | 1 (85 °C) | 155,8 | 95,3 | 83,9 | 3,1 | 1,64 | 2,77 |
| B3 | Toluol | 3 | 4 (110 °C) | 0 | 161,9 | 95,9 | 88,7 | 3,9 | 1,09 | 1,93 |
| | | | | | | | | | | |
| C1 | Chlorbenzol | 3 | 4 (85-90 °C) | 12 (85-90°C) | 150,2 | 88,2 | 75,6 | 8,3 | 5,87 | 9,65 |
| C2 | Chlorbenzol | 3 | 4 (85-90 °C) | 12 (85-90°C) | 143,9 | 89,9 | 73,9 | 10,0 | 5,68 | 8,94 |
| C3 | Chlorbenzol | 3 | 4 (85-90 °C) | 0 | 153,6 | 96,7 | 84,8 | 8,1 | 1,57 | 2,64 |

| | | | | | | | | | | |
|---|---|---|---|---|---|---|---|---|---|---|
| * Faktor 1,5 | | | | | | | | | | |

Die vorstehende Tabelle vergleicht das erfindungsgemäß eingesetzte Lösungsmittel Isopropylacetat mit den Lösungsmitteln Toluol und Chlorbenzol bei einheitlichem Einsatz von Thionylchlorid als Chlorierungsmittel und bei jeweils mehrstündiger Reaktionszeit und bei in einem engen Bereich liegenden Reaktionstemperaturen.

### VERGLEICH der AUSBEUTEN

Der tabellarische Vergleich belegt durch die Experimente A2, A3, A4 und B3 sowie das Experiment C3 zunächst, dass die Ausbeuten der Reaktion bei allen Lösungsmitteln höher sind, sofern kein Nachrühren der Reaktionslösung stattfindet. In der industriellen Herstellung kann ein Nachrühren der Reaktionslösung jedoch aus technischen Gründen häufig nicht vermieden werden.

So belegt die Tabelle durch die Experimente A1 und B2 sowie C1 und C2, dass bereits ein einstündiges Nachrühren der Reaktionslösung nach mehrstündiger Reaktionsdauer (d.h. nach 3,5 Stunden bis 7 Stunden Reaktionsdauer) die Ausbeute der Reaktion deutlich verschlechtert. Lediglich Experiment B1 bildet eine Ausnahme und bestätigt nicht, dass auch bereits bei nur einstündigem Nachrühren mit einer Minderung der Ausbeute gerechnet werden muss.

Weiterhin fällt beim Vergleich der verschiedenen Lösungsmittel auf, dass die Verschlechterung der Ausbeute trotz einer extrem langen Nachrührzeit von 60 Stunden (Experiment A1) bei Verwendung von Isopropylacetat als Lösungsmittel vergleichsweise gering ist und die Ausbeute dennoch bei über 90 %, nämlich bei 91.4% liegt. Im Vergleich dazu liegt die Ausbeute bei Verwendung der Lösungsmittel Toluol und Chlorbenzol in beiden Fälle unter 90 %, nämlich bei 88,7% (Experiment B3) und 84,8% (Experiment C3), wobei in den beiden zuletzt genannten Experimenten nicht nachgerührt wird. Wird bei Verwendung der Lösungsmittel Toluol und Chlorbenzol noch nachgerührt, so ist die Ausbeute noch schlechter, nämlich z.B. bei 83,9% (Experiment B2) und bei 73,9% (Experiment C2).

### VERGLEICH der DIMERBILDUNG

Als besonderer Vorteil des erfindungsgemäßen Verfahrens ist die geringe Dimerbildung bei Einsatz von Isopropylacetat als Lösungsmittel festzustellen.

Die Experimente A2 und A3 belegen im Vergleich zum Experiment A1, dass bei Einsatz von Isopropylacetat als Lösungsmittel auch eine extrem lange Nachrührzeit von 60 Stunden (Experiment A1) keinen wesentlichen Einfluss auf die unerwünschte Dimerbildung hat.

Eine Ausnahme bildet das Experiment A4 mit einem Wert von 0,67. Allerdings ist der Wert von 0,67 immer noch deutlich geringer als die entsprechenden Werte bei Einsatz von Toluol und Chlorbenzol als Lösungsmittel. Die entsprechenden Werte bei Einsatz von Toluol und Chlorbenzol liegen im Bereich 1,85 bis 9,65. Auffallend hoch ist Dimerbildung bei Einsatz von Chlorbenzol als Lösungsmittel bei mehrstündigem Nachrühren (vgl. Experimente C1 und C2).

Zusammenfassend kann in Bezug auf Tabelle 1 festgehalten werden, dass beim vergleichenden Einsatz eines der erfindungsgemäßen Lösungsmittel, nämlich bei Einsatz von Isopropylacetat im Vergleich zu Toluol und Chlorbenzol, die Verbesserung der Ausbeute unerwartet hoch ist und zugleich die unerwünschte Dimerbildung überraschend niedrig ist.

Somit ist der Einsatz von Isopropylacetat in zweifacher Hinsicht vorteilhaft und ermöglicht ein robustes Verfahren, das für den industriellen Einsatz aufgrund des Einsparpotentials verschiedener Ressourcen auch aus wirtschaftlichen Gründen besonders geeignet und vorteilhaft ist.

In der oben definierten Gruppe der aprotischen und zugleich polaren Lösungsmittel sind bestimmte aprotische polare Lösungsmittel bei der Durchführung des erfindungsgemäßen Verfahrens bevorzugt.

Bevorzugte aprotische polare Lösungsmittelklassen sind offenkettige Ketone, cyclische Ketone, Ester, Amide, Nitrile oder Ether, die jeweils unsubstituiert oder substitiuiert sind, wobei die jeweiligen Lösungsmittelmoleküle unsubstituiert oder durch einen oder mehrere Substituenten, ausgewählt aus der aus
- Fluor, Chlor, Brom, Iod, und
- (C₁-C₄)-Alkyl
bestehenden Gruppe, substituiert sind.

Bevorzugt erfolgt die Umsetzung der Edukte der Formel (III) und der Formel (IV) in einem Gemisch von aprotisch polaren Lösungsmittel mit einem Molekulargewicht von jeweils unter 200 erfolgt, wobei das Gemisch mindestens zwei oder mehreren Lösungsmitteln ausgewählt aus der Gruppe bestehend aus Cyclohexanon, Methylisobutylketon, Di-isobutylketon, Ethylacetat, Propylacetat, Butylacetat, Isopropylacetat, Isobutylacetat, Ethylpropionat, Ethylbutyrat, Propylpropionat, Isopropyl propionat, Di-alkylacetamid, Cycloalkylacetamid, Acetonitril, Propionitril, Butyronitril, Valeronitril, Methyl-tert.-Butylether, Tetrahydrofuran und Methyltetrahydrofuran, umfasst.

Besonders bevorzugt erfolgt die Umsetzung der Edukte der Formel (III) und der Formel (IV) ausschließlich in einem spezifischen Lösungsmittel, wobei das Lösungsmittel ausgewählt ist aus der Gruppe bestehend aus Cyclohexanon, Methylisobutylketon, Di-isobutylketon, Ethylacetat, Propylacetat, Butylacetat, Isopropylacetat, Isobutylacetat, Ethylpropionat, Ethylbutyrat, Propylpropionat, Isopropyl propionat, Di-alkylacetamid, Cycloalkylacetamid, Acetonitril, Propionitril, Butyronitril, Valeronitril, Methyl-tert.-Butylether, Tetrahydrofuran und Methyltetrahydrofuran. Am meisten bevorzugt sind dabei die beiden Lösungsmittel Isopropylacetat und Isobutylacetat.

Es ist ebenso möglich, dass die Umsetzung der Edukte der Formel (III) und der Formel (IV) nicht in nur einem spezifschen aprotischen polaren Lösungsmittel, sondern in einer Mischung aus verschiedenen Lösungsmitteln, erfolgt. In diesem Fall enthält die Lösungsmittelzusammensetzung mindestens zwei Lösungsmittel, die jeweils ausgewählt sind aus der Gruppe bestehend aus Cyclohexanon, Methylisobutylketon, Di-isobutylketon, Ethylacetat, Propylacetat, Butylacetat, Isopropylacetat, Isobutylacetat, Ethylpropionat, Ethylbutyrat, Propylpropionat, Isopropyl propionat, Di-alkylacetamid, Cycloalkylacetamid, Acetonitril, Propionitril, Butyronitril, Valeronitril, Methyl-tert.-Butylether, Tetrahydrofuran und Methyltetrahydrofuran.

Ganz besonders bevorzugt erfolgt die Umsetzung der Edukte der Formel (III) und der Formel (IV) in einem Lösungsmittel, welches ausgewählt ist aus der Gruppe der Carbonsäureester oder in einer Lösungsmittelzusammensetzung mit mindestens zwei aus der Gruppe der Carbonsäureester ausgewählten Lösungsmitteln.

Ein wesentlicher Vorteil der Verwendung von Carbonsäureestern als Lösungsmittel besteht auch darin, dass deren Rückgewinnung weniger aufwendig ist als die Rückgewinnung eines aromatischen Lösungsmittels, wie z.B. die Rückgewinnung von Chlorbenzol oder Toluol. Durch die verbesserte Lösungsmittelrückgewinnung werden im Interesse der Nachhaltigkeit die Abfallmengen insgesamt deutlich reduziert.

Am meisten wiegt jedoch der Vorteil, dass die Bildung von Dimeren bei Durchführung der Reaktion in einem Carbonsäureester oder einem Carbonsäureestergemisch als Lösungsmittel vermieden wird.

Am meisten bevorzugt ist die Umsetzung der Edukte der Formel (III) und der Formel (IV) in einem Lösungsmittel welches ausgewählt ist aus Gruppe bestehend aus Isopropylacetat, Isobutylacetat und Ethylpropionat oder in einer Lösungsmittelzusammensetzung mit mindestens zwei aus der Gruppe bestehend aus Isopropylacetat, Isobutylacetat und Ethylpropionat ausgewählten Lösungsmitteln.

Falls die Umsetzung in nur einem Lösungsmittel erfolgt, so ist Isopropylacetat das am besten geeignete Lösungsmittel. Bei Einsatz von Isopropylacetat als Lösungsmittel kann die Verbindung der Formel (II) durch Umsetzung der Edukte der Formel (III) und der Formel (IV) mit besonders guter Ausbeute und guter Qualität hergestellt werden.

Als weiterer Vorteil von Isopropylacetat wurde außerdem festgestellt, dass bei Verwendung von Isopropylacetat als Lösungsmittel die Rückgewinnung des bei der Reaktion im Überschuss eingesetzten Chlorierungsmittels besonders effizient ist. Dies ist im Interesse der Umwelt und ist auch aus Kostengründen bedeutsam.

Als Chlorierungsmittel sind im Zusammenhang mit dem erfindungsgemäßen Verfahren prinzipiell alle dem Fachmann als geeignet bekannten Chlorierungsmittel einsetzbar, wobei auch denkbar ist, dass ein aus mehreren verschiedenen Chlorierungsmitteln bestehendes Gemisch eingesetzt wird.

Bevorzugte Chlorierungsmittel sind ausgewählt aus der Gruppe der Schwefel oder Phosphor basierten Chlorierungsmittel. Dazu gehören Thionylchlorid, Phosphoroxychlorid oder Phosphorpentachlorid, oder auf Kohlenstoff basierte Chlorierungsmittel, wie Oxalylchloride oder Phosgen. Letztere sind verwendbar, um eine Carboxylsäure in ein entsprechendes Säurechlorid umzuwandeln.

Besonders bevorzugt als Chlorierungsmittel sind Cl₂, SO₂Cl₂, SOCl₂ (Thionylchlorid), N-Chlorsuccinimid, wobei das Chlorierungsmittel Thionylchlorid am meisten bevorzugt ist. Denkbar ist auch der Einsatz eines Gemisches bestehend aus mindestens zwei der vorgenannten Chlorierungsmittel.

Weitere, jeweils alternativ oder in Kombination, einsetzbare Chlorierungsmittel sind Siliciumtetrachlorid, Trichlormethylsilan, Dichlormethylsilan, Trichlorphenylsilan, Aluminiumtrichlorid, Bortrichlorid, Titantetrachlorid, Zinntetrachlorid, Zinkdichlorid oder Wismutttrichlorid, bzw. ein Gemisch derselben. Eingesetzt werden können auch Gemische aus Halogensilanen und Aluminiumtrichlorid oder Zinkdichlorid, z.B. Gemische aus Siliciumtetrachlorid und Aluminiumtrichlorid, wobei Aluminiumtrichlorid oder Zinkdichlorid als Katalysator dienen und in Mengen von 1 bis 3 Gew.% bezogen auf Siliciumtetrachlorid eingesetzt werden.

Bei der Durchführung des erfindungsgemäßen Verfahrens setzt man pro Äquivalent der Formel (III oder IV) zwischen 2,5 und 3,0 Äquivalente an austauschbaren Chloratomen eines Chlorierungsmittels, bzw. Chlorierungsmittelgemisches, ein. Bevorzugt werden 2,5 Äquivalente Thionylchlorid eingesetzt.

Die Verbindungen der Formel (III) und (IV) werden in äquimolaren Mengen eingesetzt.

Der Einsatz eines Katalysators ist bei der erfindungsgemäßen Reaktion vorteilhafter Weise nicht erforderlich.

Die Reaktionstemperaturen können bei der Durchführung des erfindungsgemäßen Verfahrens in den nachfolgend bestimmten Bereichen variiert werden. Im Allgemeinen arbeitet man bei Temperaturen im Bereich von 20 °C bis 90 °C. Bevorzugt sind Temperaturen im Bereich von 40 °C bis 90 °C. Besonders bevorzugt sind Temperaturen im Bereich von 80 °C bis 90 °C.

Das erfindungsgemäße Verfahren wird im Allgemeinen unter Normaldruck durchgeführt. Es ist jedoch auch möglich unter erhöhtem oder vermindertem Druck zu arbeiten. Der bevorzugte Druckbereich für die Durchführung der erfindungsgemäßen Reaktion liegt zwischen 0,1 bar und 10 bar.

### SYNTHESEBEISPIEL

### Herstellung von Cyprosulfamide ausgehend von 4-[[(2-Methoxybenzoyl)amino]-sulfonyl]benzoylchlorid

1,8 I/eq Wasser und 0,10 eq. (Äquivalente) Cyclopropylamin werden bei ca. 20°C vorgelegt. (Es werden maximal 0,10 eq. Cyclopropylamin (CPA), d.h. ≤ 0,10 eq. Cyclopropylamin, vorgelegt. Das vorgelegte CPA kann optional aus der Wiedergewinnung des vorherigen Ansatzes stammen.)

4[[Methoxybenzoyl)amino]sulfonyl]benzoylchlorid (Amidchlorid) wird bei 20° C - 35° C über die Zeitdauer von 4h (4 Stunden) portionsweise (24 Portionen) zugegeben. Parallel werden dazu 1,1 eq. CPA so dosiert, dass der pH auf 9,0-9,6 gehalten wird. Nach ca. 20% der Amidchloridmenge ist die CPA-Menge komplett eindosiert.

Danach wird weiter parallel 2 eq. 32%iger NaOH so dosiert, dass der pH auf 9,2-9,8 gehalten wird. Nach Zugabe von ca. 2/3 der Amidchloridmenge fällt das Produkt aus.

Nach vollständiger Amidchlorid- und NaOH-Zugabe wird die Suspension in 1h auf 80 °C erwärmt (dies ergibt eine trübe Lösung).

Dann wird der Überschuss CPA bei 80°C und 400-300 mbar abdestilliert (Das CPA/Wasser-Gemisch kann wieder erneut eingesetzt werden).

Zur Fällung des Produktes (zur Verbesserung der Pumpbarkeit des Produktes) wird noch Wasser zugegeben und bei 80°C mit ca. 1 eq. 10%iger Salzsäure innerhalb von 3h auf pH 5,8 - 6,2 angesäuert.

Die Suspension wird 30 Minuten bei 80°C und ca. pH6 nachgerührt und dann auf 20° C bis 30°C abgekühlt. Dabei wird der pH-Wert mit 10%iger Salzsäure auf ca.pH 6 nachgestellt. Die Suspension wird über eine Nutsche filtriert. Der Nutschkuchen wird mit Wasser gewaschen und trocken gedrückt. Der Feststoff bei 45°C und kleiner 50 mbar getrocknet. Die Ausbeute beträgt 98,7% der Theorie (Gehalt 98,8% (HPLC gegen Standard)).

## Patentansprüche

1. Verfahren zur Herstellung von Verbindungen der Formel (Ia) 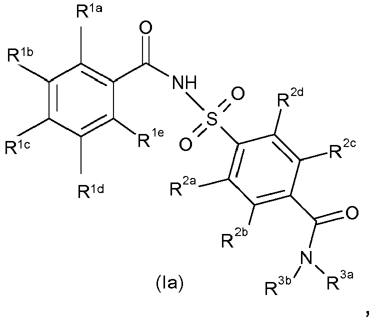 worin
R^{1a} bis R^{1e} und R^{2a} bis R^{2d} jeweils unabhängig voneinander ausgewählt sind aus der Gruppe bestehend aus Wasserstoff, Halogen, (C₁-C₆)-Alkyl, (C₁-C₆)-Halogenalkyl, (C₁-C₆)-Alkoxy, (C₃-C₇)-Cycloalkyl, (C₁-C₆)-Alkylthio, (C₃-C₇)-Cycloalkylthio, S(O)_{q}-(C₁-C₆)-Alkyl mit q = 0, 1 oder 2, (C₁-C₆)-Alkylcarbonyl, -CO-aryl, Cyano und Nitro oder in welcher jeweils zwei benachbarte Reste R^{1a} bis R^{1e} einen -O-CH₂CH₂- Rest bilden, und
R^{3a} ausgewählt ist aus der Gruppe bestehend aus Wasserstoff und den nachgenannten Resten (C₁-C₆)-Alkyl, (C₁-C₆)-Halogenalkyl, (C₃-C₇)-Cycloalkyl, (C₁-C₆)-Alkoxy, (C₁-C₆)-Alkylthio, (C₃-C₇)-Cycloalkylthio, -(CH2)p-Heterocyclyl, wobei diese jeweils unsubstituiert sind oder substituiert sind durch einen oder mehrere Substituenten ausgewählt aus der Gruppe bestehend aus Halogen, (C₁-C₆)-Alkyl, (C₁-C₆)-Halogenalkyl, (C₁-C₆)-Alkoxy, Cyano und Nitro, und
R^{3b} ausgewählt ist aus der Gruppe bestehend aus Wasserstoff und den nachgenannten Resten (C₁-C₆)-Alkyl, (C₂-C₆)-Alkenyl, (C₂-C₆)-Alkinyl, (C₁-C₆)-Alkoxy, (C₂-C₆)-Alkenyloxy, -(CH2)p-Heterocyclyl, wobei diese jeweils unsubstituiert sind oder substituiert sind durch einen oder mehrere Substituenten ausgewählt aus der Gruppe bestehend aus Halogen, (C₁-C₄)-Alkoxy und (C₁-C₄)-Alkylthio, oder
R^{3a} und R^{3b} gemeinsam mit dem verbindenden Stickstoffatom einen 3- bis 8-gliedrigen, gesättigten oder ungesättigten Ring ausbilden
durch Umsetzung einer Verbindung der Formel (II) 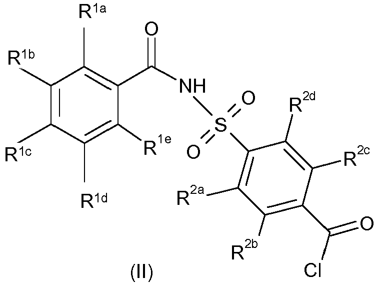 mit einem Amin der Formel R^{3a}R^{3b}NH, worin die Reste R^{3a} und R^{3b} wie oben definiert sind, in wässriger Lösung, wobei zur Regulation des pH-Werts
- bei Reaktionsbeginn ein Teil des Amins der Formel R^{3a}R^{3b}NH in Wasser vorgelegt wird, und
- während des Reaktionsverlaufs die verbleibende Menge des Amins der Formel R^{3a}R^{3b}NH dem Reaktionsgemisch in einem oder in mehreren Schritten zugegeben wird.

2. Verfahren zur Herstellung von Verbindungen der Formel (Ia) nach Anspruch 1, **dadurch gekennzeichnet, dass** R^{1a} bis R^{1e} und R^{2a} bis R^{2d} unabhängig voneinander ausgewählt sind aus der Gruppe bestehend aus
Wasserstoff, Fluor, Chlor, Brom, und
(C₁-C₆)-Alkoxy.

3. Verfahren zur Herstellung von Verbindungen der Formel (Ia) nach Anspruch 2, **dadurch gekennzeichnet, dass** R^{1a} ausgewählt ist aus der Gruppe bestehend aus unsubstituierten (C₁-C₄)-Alkoxyresten und R^{1b} bis R^{1e} sowie R^{2a} bis R^{2d} jeweils Wasserstoff sind.

4. Verfahren zur Herstellung von Verbindungen der Formel (la) nach Anspruch 3, **dadurch gekennzeichnet, dass** R^{1a} für Methoxy (-O-CH₃) steht, und R^{1b} bis R^{1e} sowie R^{2a} bis R^{2d} jeweils für Wasserstoff stehen.

5. Verfahren zur Herstellung eines *N*-[4-(cyclopropylcarbamoyl)phenylsulfonyl]-2-methoxy-benzamid nach einem der Ansprüche 1 bis 4, **dadurch gekennzeichnet dass**, in der Formel (II)
- R^{1a} für Methoxy (-O-CH₃) sowie R^{1b} bis R^{1e} sowie R^{2a} bis R^{2d} jeweils für Wasserstoff stehen, und in dem
- Amin der Formel R^{3a}R^{3b}NH der Rest R^{3a} für Cyclopropyl und R^{3b} für Wasserstoff steht.

6. Verfahren zur Herstellung einer Verbindung der Formel (Ia) nach einem der Ansprüche 1 bis 5, **dadurch gekennzeichnet, dass** bei Reaktionsbeginn jene Menge des Amins der Formel R^{3a}R^{3b}NH in Wasser vorgelegt wird, die erforderlich ist, um die Einstellung des pH-Werts der Reaktionsvorlage auf maximal 11,5 (pH ≤ 11,5) zu bewirken.

7. Verfahren zur Herstellung einer Verbindung der Formel (Ia) nach Anspruch 6, **dadurch gekennzeichnet, dass** die Zugabe der bei Reaktionsbeginn nicht vorgelegten Aminmenge des Amins der Formel R^{3a}R^{3b}NH in mehreren Schritten erfolgt, so dass der pH-Wert während der Zugabe im Bereich von 7 bis 10,5 liegt (pH = 7 - 10,5).

8. Verfahren zur Herstellung einer Verbindung der Formel (Ia) nach Anspruch 7, **dadurch gekennzeichnet, dass** die Zugabe der bei Reaktionsbeginn nicht vorgelegten Aminmenge des Amins der Formel R^{3a}R^{3b}NH in mehreren Schritten erfolgt, so dass der pH-Wert während der Zugabe im Bereich von 9 bis 10,5 liegt (pH = 9 bis 10,5).

9. Verfahren zur Herstellung einer Verbindung der Formel (Ia) nach Anspruch 6, 7 oder 8, **dadurch gekennzeichnet, dass** dem Reaktionsgemisch zur Vermeidung des Abfallens des pH-Wertes unter 8 eine Base, ausgewählt aus der Gruppe bestehend aus NaOH, KOH, Ca(OH)₂ und der Gruppe der tertiären Amine, zugegeben wird.

10. Verfahren zur Herstellung einer Verbindung der Formel (la) nach Anspruch 9, **dadurch gekennzeichnet, dass** durch Zugabe der Base NaOH der pH-Wert im Bereich von 9 bis 10 (pH = 9 bis 10) gehalten wird.

## Claims

1. Process for preparing compounds of the formula (Ia) 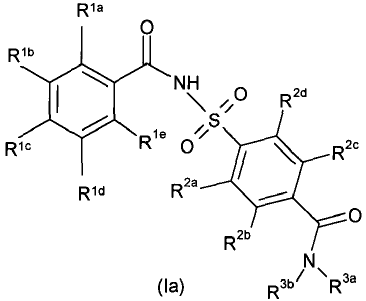 in which
R^{1a} to R^{1e} and R^{2a} to R^{2d} are each independently selected from the group consisting of hydrogen, halogen, (C₁-C₆)-alkyl, (C₁-C₆)-haloalkyl, (C₁-C₆)-alkoxy, (C₃-C₇)-cycloalkyl, (C₁-C₆)-alkylthio, (C₃-C₇)-cycloalkylthio, S(O)_{q}-(C₁-C₆)-alkyl with q = 0, 1 or 2, (C₁-C₆)-alkylcarbonyl, -CO-aryl, cyano and nitro or in which two adjacent R^{1a} to R^{1e} radicals in each case form a -O-CH₂CH₂- radical, and
R^{3a} is selected from the group consisting of hydrogen and the following radicals: (C₁-C₆)-alkyl, (C₁-C₆)-haloalkyl, (C₃-C₇)-cycloalkyl,
(C₁-C₆)-alkoxy, (C₁-C₆)-alkylthio, (C₃-C₇)-cycloalkylthio, -(CH2)p-heterocyclyl, where these are each unsubstituted or substituted by one or more substituents selected from the group consisting of halogen, (C₁-C₆)-alkyl, (C₁-C₆)-haloalkyl, (C₁-C₆)-alkoxy, cyano and nitro, and
R^{3b} is selected from the group consisting of hydrogen and the following radicals: (C₁-C₆)-alkyl, (C₂-C₆)-alkenyl, (C₂-C₆)-alkynyl, (C₁-C₆)-alkoxy, (C₂-C₆)-alkenyloxy, -(CH2)p-heterocyclyl, where these are each unsubstituted or substituted by one or more substituents selected from the group consisting of halogen, (C₁-C₄)-alkoxy and (C₁-C₄)-alkylthio, or
R^{3a} and R^{3b} together with the connecting nitrogen atom form a 3- to 8-membered, saturated or unsaturated ring,
by reacting a compound of the formula (II) 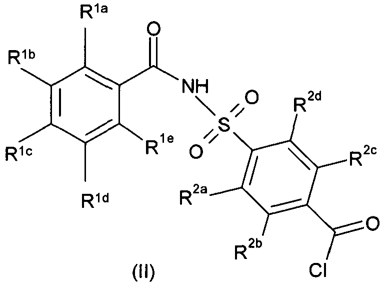 with an amine of the formula R^{3a}R^{3b}NH in which the R^{3a} and R^{3b} radicals are each as defined above, in aqueous solution, wherein the pH is regulated by
- initially charging a portion of the amine of the formula R^{3a}R^{3b}NH in water at the start of the reaction, and
- adding the remaining amount of the amine of the formula R^{3a}R^{3b}NH to the reaction mixture over the course of the reaction in one or more steps.

2. Process for preparing compounds of the formula (Ia) according to Claim 1, **characterized in that** R^{1a} to R^{1e} and R^{2a} to R^{2d} are independently selected from the group consisting of
hydrogen, fluorine, chlorine, bromine, and (C₁-C₆)-alkoxy.

3. Process for preparing compounds of the formula (Ia) according to Claim 2, **characterized in that** R^{1a} is selected from the group consisting of unsubstituted (C₁-C₄)-alkoxy radicals and R^{1b} to R^{1e} and R^{2a} to R^{2d} are each hydrogen.

4. Process for preparing compounds of the formula (Ia) according to Claim 3, **characterized in that** R^{1a} is methoxy (-O-CH₃), and R^{1b} to R^{1e} and R^{2a} to R^{2d} are each hydrogen.

5. Process for preparing an N-[4-(cyclopropylcarbamoyl)phenylsulfonyl]-2-methoxybenzamide according to one or more of Claims 1 to 4, **characterized in that**, in the formula (II),
- R^{1a} is methoxy (-O-CH₃) and R^{1b} to R^{1e} and R^{2a} to R^{2d} are each hydrogen and, in the
- amine of the formula R^{3a}R^{3b}NH, the R^{3a} radical is cyclopropyl and R^{3b} is hydrogen.

6. Process for preparing a compound of the formula (Ia) according to any of Claims 1 to 5, **characterized in that**, the amount of the amine of the formula R^{3a}R^{3b}NH which is initially charged in water at the start of the reaction is that which is required to bring about the adjustment of the pH of the initial reaction charge to a maximum of 11.5 (pH ≤ 11.5).

7. Process for preparing a compound of the formula (Ia) according to Claim 6, **characterized in that** the addition of the amount of the amine of the formula R^{3a}R^{3b}NH which is not initially charged at the start of the reaction is effected in several steps, such that the pH during the addition is in the range from 7 to 10.5 (pH = 7-10.5).

8. Process for preparing a compound of the formula (Ia) according to Claim 7, **characterized in that** the addition of the amount of the amine of the formula R^{3a}R^{3b}NH which is not initially charged at the start of the reaction is effected in several steps, such that the pH during the addition is in the range from 9 to 10.5 (pH = 9 to 10.5).

9. Process for preparing a compound of the formula (Ia) according to Claim 6, 7 or 8, **characterized in that** a base selected from the group consisting of NaOH, KOH, Ca(OH)₂ and the group of the tertiary amines is added to the reaction mixture in order to prevent the pH from dropping below 8.

10. Process for preparing a compound of the formula (Ia) according to Claim 9, **characterized in that** addition of the base NaOH keeps the pH in the range from 9 to 10 (pH = 9 to 10).

## Revendications

1. Procédé de fabrication de composés de la formule (Ia) 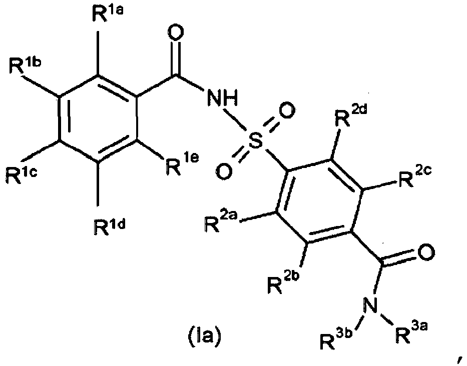 dans laquelle
R^{1a} à R^{1e} et R^{2a} à R^{2d} sont chacun choisis indépendamment les uns des autres dans le groupe constitué par hydrogène, halogène, alkyle en (C₁-C₆), halogénoalkyle en (C₁-C₆), alcoxy en (C₁-C₆), cycloalkyle en (C₃-C₇), alkylthio en (C₁-C₆), cycloalkylthio en (C₃-C₇), S(O)_{q}-alkyle en (C₁-C₆) avec q = 0, 1 ou 2, alkylcarbonyle en (C₁-C₆), -CO-aryle, cyano et nitro, ou dans laquelle deux radicaux R^{1a} à R^{1e} voisins forment à chaque fois un radical -O-CH₂-CH₂-, et
R^{3a} est choisi dans le groupe constitué par hydrogène et les radicaux indiqués ci-après : alkyle en (C₁-C₆), halogénoalkyle en (C₁-C₆), cycloalkyle en (C₃-C₇), alcoxy en (C₁-C₆), alkylthio en (C₁-C₆), cycloalkylthio en (C₃-C₇), -(CH₂)ₚ-hétérocyclyle, ceux-ci étant à chaque fois non substitués ou substitués par un ou plusieurs substituants choisis dans le groupe constitué par halogène, alkyle en (C₁-C₆), halogénoalkyle en (C₁-C₆), alcoxy en (C₁-C₆), cyano et nitro, et
R^{3b} est choisi dans le groupe constitué par hydrogène et les radicaux indiqués ci-après : alkyle en (C₁-C₆), alcényle en (C₂-C₆), alcynyle en (C₂-C₆), alcoxy en (C₁-C₆), alcényloxy en (C₂-C₆), -(CH₂)ₚ-hétérocyclyle, ceux-ci étant à chaque fois non substitués ou substitués par un ou plusieurs substituants choisis dans le groupe constitué par halogène, alcoxy en (C₁-C₄) et alkylthio en (C₁-C₄), ou
R^{3a} et R^{3b} forment ensemble avec l'atome d'azote qui les relie un cycle saturé ou insaturé de 3 à 8 chaînons,
par mise en réaction d'un composé de formule (II) 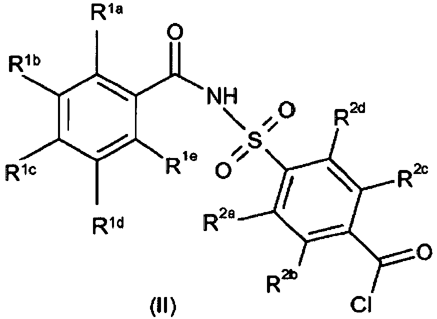 avec une amine de formule R^{3a}R^{3b}NH, les radicaux R^{3a} et R^{3b} étant tels que définis précédemment, dans une solution aqueuse, pour la régulation du pH,
- une partie de l'amine de formule R^{3a}R^{3b}NH étant chargée dans de l'eau au début de la réaction, et
- pendant le déroulement de la réaction, la quantité restante de l'amine de formule R^{3a}R^{3b}NH étant ajoutée au mélange réactionnel en une ou en plusieurs étapes.

2. Procédé de fabrication de composés de la formule (Ia) selon la revendication 1, **caractérisé en ce que** R^{1a} à R^{1e} et R^{2a} à R^{2d} sont choisis indépendamment les uns des autres dans le groupe constitué par :
hydrogène, fluor, chlore, brome, et
alcoxy en (C₁-C₆).

3. Procédé de fabrication de composés de la formule (Ia) selon la revendication 2, **caractérisé en ce que** R^{1a} est choisi dans le groupe constitué par les radicaux alcoxy en (C₁-C₄) non substitués, et R^{1b} à R^{1e}, ainsi que R^{2a} à R^{2d} représentent chacun l'hydrogène.

4. Procédé de fabrication de composés de la formule (Ia) selon la revendication 3, **caractérisé en ce que** R^{1a} représente méthoxy (-O-CH₃), et R^{1b} à R^{1e}, ainsi que R^{2a} à R^{2d} représentent chacun l'hydrogène.

5. Procédé de fabrication d'un *N*-[4-(cyclopropylcarbamoyl)phénylsulfonyl]-2-méthoxybenzamide selon l'une quelconque des revendications 1 à 4, **caractérisé en ce que**, dans la formule (II),
- R^{1a} représente méthoxy (-O-CH₃), et R^{1b} à R^{1e}, ainsi que R^{2a} à R^{2d} représentent chacun l'hydrogène, et
- dans l'amine de formule R^{3a}R^{3b}NH, le radical R^{3a} représente cyclopropyle et R^{3b} représente l'hydrogène.

6. Procédé de fabrication d'un composé de la formule (Ia) selon l'une quelconque des revendications 1 à 5, **caractérisé en ce qu'**au début de la réaction, la quantité de l'amine de formule R^{3a}R^{3b}NH qui est nécessaire pour réaliser l'ajustement du pH de la préparation de réaction à au plus 11,5 (pH ≤ 11,5) est chargée dans de l'eau.

7. Procédé de fabrication d'un composé de la formule (Ia) selon la revendication 6, **caractérisé en ce que** l'ajout de la quantité de l'amine de formule R^{3a}R^{3b}NH non chargée au début de la réaction a lieu en plusieurs étapes, de telle sorte que le pH se situe pendant l'ajout dans la plage allant de 7 à 10,5 (pH = 7 à 10,5) .

8. Procédé de fabrication d'un composé de la formule (Ia) selon la revendication 7, **caractérisé en ce que** l'ajout de la quantité de l'amine de formule R^{3a}R^{3b}NH non chargée au début de la réaction a lieu en plusieurs étapes, de telle sorte que le pH se situe pendant l'ajout dans la plage allant de 9 à 10,5 (pH = 9 à 10,5) .

9. Procédé de fabrication d'un composé de la formule (Ia) selon la revendication 6, 7 ou 8, **caractérisé en ce qu'**une base, choisie dans le groupe constitué par NaOH, KOH, Ca(OH)₂ et le groupe des amines tertiaires, est ajoutée au mélange réactionnel pour éviter la chute du pH en dessous de 8.

10. Procédé de fabrication d'un composé de la formule (la) selon la revendication 9, **caractérisé en ce que** le pH est maintenu dans la plage allant de 9 à 10 (pH = 9 à 10) par ajout de la base NaOH.
